# EUROPEAN PATENT APPLICATION

(11) **EP 2 742 966 A1**
(43) Date of publication of application: **18.06.2014**
(21) Application number: 12821937.5
(22) Date of filing: 10.07.2012
(51) Int. Cl.: A61M 29/04

(54) **APPLICATION OF WATER-ABSORPTION MATERIAL IN MEDICAL CAVITY CHANNEL EXPANDER**

(30) Priority: 10.08.2011 CN 201110235444
(71) Applicant: Liaoning Aimu Medical Science&Technology Co., Ltd., Anshan, Liaoning 114044 (CN)
(72) Inventor: WU, Xiaofeng, Anshan, Liaoning 114044 (CN); SHI, Fengyang, Anshan, Liaoning 114044 (CN)
(74) Representative: Bergh, Johanna
(86) International application number: PCT/CN2012/078423
(87) International publication number: WO 2013/020435

(57) **Abstract**

This invention provides a medical cavity dilator made of water-absorbent resin. This cavity dilator has relatively great improvements in water absorbability and the rate of absorbing water, especially in the rate of absorbing water. It can greatly reduce patients' waiting time and applicable to dilation for all cavity operations. Besides, the surface of the cavity dilator exhibits the effect of super-slipperiness after absorbing water, and thus can successfully enter a cavity without the need of applying a lubricator thereon.

## Description

### FIELD OF THE INVENTION

The present invention relates to a medical dilator, more specifically, to the use of a water-absorbent material in a dilator for natural cavity.

### BACKGROUND OF THE INVENTION

The present invention is put forward on the basis of the following two aspects. I. Water-absorbent resin is a sort of functional polymeric materials having a large amount of hydrophilic groups. Because the water-absorbent resin has high liquid absorbability, high rate of absorbing liquid and high liquid retentivity, it is widely used in various fields such as agriculture and horticulture (for example used as water-retaining agent and soil improvement agent), industry (for example used as moisture resisting agent, oil-displacing agent for oilfield, dehydrating agent for organic solvent, thickening agent, water-soluble paint, and the like), medical health (for example used as medical substrate material for sanitary towel, baby diaper, napkin, medical ice bag, ointment, cream, liniment, cataplasm, or the like, as well as made into intelligent carrier for controlling the amount, time, and space of drug release), and architecture (used for plugging water in precast slab slots in cellar, tunnel, and subway; and for solidifying mud in municipal waste water treatment and dredging).

Water-absorbent resin has such a high water-absorbent function that it can absorb water hundreds to thousands times heavier than itself. It also has good water retentivity. Once the water-absorbent resin becomes hydrogel after absorbing water and swelling, it is hard to separate water therefrom even though by pressurization. Since the high water-absorbent polymer has characteristics such as being nontoxic, being non-irritant to human body, having no side effect, and not causing blood coagulation, it is widely used in recent years, especially in the medical field. For example, it can be used for: ointment, which is for external use and comfortable in use, and has high water content; producing medical bandages and cotton balls which can absorb blood and secretion in operations and traumas and prevent suppuration; manufacturing anti-infective artificial skin through which water and medicine can pass while microorganisms can not permeate; or the like.

II. In human body, there are some natural cavities, such as vagina, uterus, oral cavity, nasal cavity, external auditory canal, lacrimal passage and the like, in which a cavity dilator is often needed when an operation is carried out.

As an example, uterus is taken. When operations on uterus or fallopian tube are carried out, in most cases, surgical instruments, which enter uterus or fallopian tube through cervix, are needed. The size of the opening of cervix in normal state is relatively small. If a surgical instrument having an external diameter less than 5 mm enters, it is not necessary to dilate the cervix. However, currently for most operations, the external diameters of instruments (for example: some intrauterine device-inserting devices, miscarriage pipets, hysteroscopic instruments, etc.) are more than 5 mm, and therefore it is necessary to dilate the cervix when using such instruments. Furthermore, for some operations, which do not need the help of instruments (such as childbirth and induced abortion), it is also necessary to dilate the cervix. General cervix dilators used in hospitals are made of metallic materials and reusable, and dilate the cervix mechanically and forcibly. Although cervical dilators used as supporting equipments for products such as miscarriage pipets and the like are made of disposable plastic materials, they also dilate the cervix mechanically and forcibly.

People are also attempting dilators based on other dilation principles. For example, ZL90210761.5 discloses a gelatin-made cervical dilation suppository applicable to childbirth and induced abortion, which, by means of gradual dilation by absorbing water, alleviates the pain caused by mechanical and forcible dilation by conventional cervical dilators. A cervix-dilating laminaria tent available in the market is applicable to childbirth, artificial abortion, and hysteroscopic operations, and also adopts gradual dilation by absorbing water. However, the two materials mentioned above has a low rate of absorbing water of from a few hours to 24 hours, and thus the patient's waiting time is relatively long. Besides, it takes only a relatively short operation time in the intrauterine device-inserting operation, and thus it seems that the two materials mentioned above are not applicable.

Base on the above-mentioned background, report about the use of water-absorbent material in medical dilator has not been found heretofore.

### SUMMARY OF THE INVENTION

The object of the present invention is to use well-known water-absorbent materials in medical cavity dilators.

To achieve the above object, the medical cavity dilator of the present invention is made of a water-absorbent resin.

Said cavity dilator, wherein the water-absorbent resin of the cavity dilator is in a dry or semi-dry state before the cavity dilator is used.

Said cavity dilator, wherein before being used, the cavity dilator pre-absorbs a part of medical liquid.

Said cavity dilator, wherein before being used, the cavity dilator is subjected to sterilization, and the sterilization method is sterilization by irradiation.

The cavity dilator provided by the present invention has relatively great improvements in water absorbability and the rate of absorbing water, especially in the rate of absorbing water. It can greatly reduce patients' waiting time and applicable to dilation for all of the above-mentioned cavity operations. And the surface of the cavity dilator of the present invention exhibits the effect of super-slipperiness after absorbing water, and thus can successfully enter a cavity without the need of applying a lubricator thereon.

### BRIEF DESCRIPTION OF THE DRAWINGS

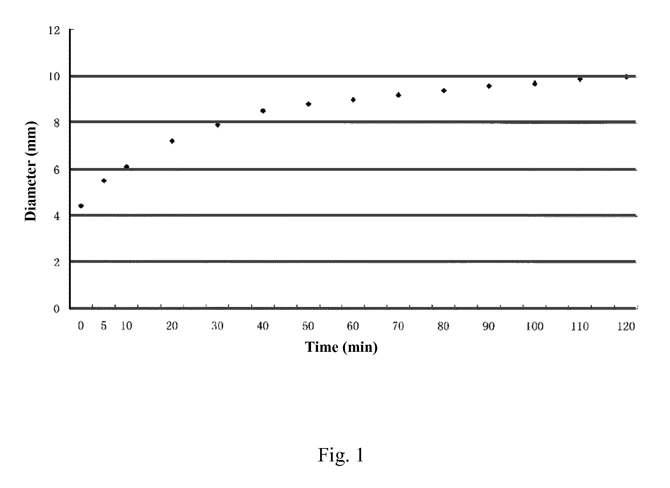

Fig. 1 is a schematic diagram showing the change in size of a cavity dilator made of a water-absorbent resin before and after absorbing water.

### DETAILED DESCRIPTION OF THE INVENTION

The water-absorbent resin adopted by the present invention is of solid structure and basically formed by cross-linking and polymerizing a substrate A and a cross-linker B.

The substrate A is an unsaturated organic acid comprising one or more C=C, C≡C, N=N, C=S, N=S, S=S, C=N or cyclic structure(s), or a derivative thereof.

The cross-linker B is an organic substance comprising two or more C=C, C≡C, N=N, C=S, N=S, S=S, C=N or cyclic structures.

The organic acid is carboxylic acid, sulfonic acid, sulfinic acid or thionothiolic acid.

The derivative of the organic acid is acyl halide, acidamide, acid anhydride or ester.

The organic acid is partly or completely neutralized by alkali before polymerization.

The alkali is potassium hydroxide, sodium hydroxide or lithium hydroxide.

The way of the polymerization is polymerization by heating, polymerization by adding initiator or irradiation polymerization.

The sterilization method for the cavity dilator is sterilization by irradiation.

Before use, the cavity dilator pre-absorbs in vitro a part of medical liquid. The medical liquid can be pure water (distilled water or sterile water for injection), a solution (physiological saline, glucose solution, or the like), or a liquid medicine playing an auxiliary role (anaesthetic, anti-inflammatory and analgetic drug, or the like).

Since the water-absorbent resin uses organic acid and strong alkali as raw materials to begin with, it still exhibits certain acidity or alkalinity after being partially neutralized. But after it is used in a human body, the main injury caused by the medical material to the human body lies in that it releases chemical substances into the human body. However, after in contact with human body, the cavity dilator of the present invention has a liquid-absorbent performance much superior to its performance of releasing substances to the human body. Therefore, no matter whether the cavity dilator itself is more acidic or more alkaline, it will not cause injury to the human body. In the 24-hour cytotoxicity test carried out on the samples produced in Example 1 of the invention, the relative cell proliferation ratio could be 80% or more, which achieved level 1. Therefore, the risk is acceptable as far as a short-time-contact medical device (period of time of contacting the human body is shorter than 24 hours) is concerned.

Sterilization is needed, before the material useful in the cavity dilation is used. Application of sterilization by irradiation can facilitate farther polymerization and crosslink of the water-absorbent resin. Even if the polymerization process is incomplete, it can be redeemed by the sterilization by irradiation.

A conventional cavity dilator is made of metal or plastic, and encounters a certain resistance force when entering a cavity. Generally, the cavity dilator needs the help of lubricator to enter. However, the surface of the water-absorbent resin exhibits the effect of super-slipperiness after absorbing water, and thus it can successfully enter a cavity without the need of applying a lubricator thereon.

### Example 1

A common water-absorbent resin (Super Absorbent Polymer, SAP) was taken as an example. A dilator appropriate to a natural cavity in human body was prepared by injecting the water-absorbent resin into a container or a mold, subsequently performing a water bath at a constant temperature ranging from 40°C to 80°C for 30 min to 3 h followed by moulding, or, by extruding the water-absorbent resin via an extruding device. Depending on various formulations, the prepared cavity dilator's diameter shrank to 50%-90% of the initial diameter thereof after being dried. Taking a shrinkage ratio proportion of 80% as an example, when the diameter of the mold cavity was 5.5 mm, the diameter of the cavity dilator was 4.4 mm after shrinking. The diameter came back to 5.5 mm after the cavity dilator had absorbed water for circa 5 min, which is applicable to intrauterine device-inserting operation. And the diameter could be more than 10 mm after the cavity dilator had absorbed water for circa 2 h, which is applicable to operations for childbirth, artificial abortion and the like. Compared with the products made of gelatin or laminaria, such a cavity dilator greatly cuts down the required time for dilation and can reduce patients' waiting time. The change in size of the cavity dilator of the present invention during the water absorption is shown in Fig. 1.

## Claims

1. A medical cavity dilator, made of a water-absorbent resin.

2. The cavity dilator according to claim 1, wherein the water-absorbent resin of the cavity dilator is in a dry or semi-dry state before the cavity dilator is used.

3. The cavity dilator according to claim 1, wherein before being used, the cavity dilator pre-absorbs a part of medical liquid.

4. The cavity dilator according to claim 1, wherein before being used, the cavity dilator is subj ected to sterilization.

5. The cavity dilator according to claim 1 or 4, wherein before being used, the cavity dilator is subj ected to sterilization by irradiation.
